# EUROPÄISCHE PATENTSCHRIFT

(11) **EP 0 766 734 B1**
(45) Veröffentlichungstag und Bekanntmachung des Hinweises auf die Patenterteilung: **27.08.2003**
(21) Anmeldenummer: 95924918.6
(22) Anmeldetag: 23.06.1995
(51) Int. Cl.: C12N 9/96, G01N 33/532, G01N 33/58, G01N 33/577

(54) **Verfahren zur Stabilisierung von hydrolyseempfindlichen Molekülen**
method of stabilizing molecules which are sensitive to hydrolysis
PROCEDE DE STABILISATION DE MOLECULES SENSIBLES A L'HYDROLYSIS

(30) Priorität: 24.06.1994 DE 4421907; 31.03.1995 DE 19511940
(43) Veröffentlichungstag der Anmeldung: 09.04.1997
(73) Patentinhaber: Dade Behring Marburg GmbH, 35001 Marburg (DE)
(72) Erfinder: NEUENHOFER, Stephan, D-35037 Marburg (DE); KÄSMARKER, Reinhard, D-35041 Marburg (DE); WALTER, Götz, D-35274 Gro seelheim (DE); HARTHUS, Hans-Peter, D-35041 Marburg-Wehrda (DE); NAU, Günther, D-35043 Marburg-Schröck (DE); SKRZIPCZYK, Heinz-Jürgen, D-63263 Neu-Isenburg (DE); MOLZ, Peter, D-55118 Mainz (DE); MADRY, Norbert, D-35041 Marburg (DE)
(86) Internationale Anmeldenummer: EP9502446
(87) Internationale Veröffentlichungsnummer: WO96000284

(56) Entgegenhaltungen:
- EP-A- 0 487 301
- WO-A-93/20074
- US-A- 5 302 533
- CHEMICAL ABSTRACTS, vol. 91, no. 9, 27.August 1979 Columbus, Ohio, US; abstract no. 71265, R. D. NARGESSI ET AL. 'Use of antibodies against the label in non-separation non-isotopic immunoassay: 'indirect quenching' fluoroimmunoassay of proteins.' Seite 305; Spalte 1; & J. IMMUNOL. METHODS, Bd. 26, Nr. 4, 1979 Seiten 307-313,

## Beschreibung

Die vorliegende Erfindung betrifft ein Verfahren zur Stabilisierung von hydrolyseempfindlichen Molekülen, wobei daß das Molekül ein in einem immunologischen Nachweisverfahren verwendetes Label ist, ausgewählt aus der folgenden Liste: Acridiniumester, Acridiniumacylsulfonamid, Luminol, Isoluminol oder dessen Derivate, Dioxetan, Luciferin, Oxalsäureester und Oxalsäureamid.

Immunologische Nachweisverfahren haben in der in vitro Diagnostik eine hohe Bedeutung erlangt. Ursache dafür ist, daß sie hochspezifisch und äußerst empfindlich sind. Zudem zeichnen sich diese Verfahren durch eine einfache Handhabung aus. Die Nachweisverfahren beruhen auf der immunologischen Wechselwirkung zwischen dem nachzuweisenden Analyten und seinem Bindungspartner bzw. seinen Bindungspartnern.

Im Falle der "Sandwich-Assays" wird der Analyt von zwei verschiedenen Antikörpern sandwichartig gebunden. Einer der beiden Antikörper trägt eine Markierung, wodurch seine Konzentration und damit die des Sandwichkomplexes bestimmt werden kann. Im Falle kleiner Analyte scheidet das Sandwich-Verfahren aus, da aus sterischen Gründen nicht gleichzeitig zwei verschiedene Antikörper am Analyten binden können. Hier finden in der Regel die kompetitiven Assays Anwendung. Dabei konkurrieren der Analyt und ein synthetisches Derivat des Analyten um die Bindungsstellen des Antikörpers. Markiert wird in der Regel entweder das Analytderivat (klassisch kompetitives Verfahren) oder der Antikörper.

Gebräuchliche Markierungen sind z.B. radioaktive Isotope oder lumineszierende (fluoreszierende, phosphoreszierende, chemilumineszierende, biolumineszierende usw.) oder zur Absorption befähigte Substanzen. Das markierte Reagens (Antikörper, Analytderivat) wird im folgenden als Tracer bezeichnet.

Die Aufbewahrung des Tracers erfolgt häufig in einer für den Anwender gebrauchsfertigen wäßrigen Lösung. Da es sich in vielen Fällen bei den Labeln um nicht hydrolyseresistente Substanzen handelt, bedeutet die Aufbewahrung in wäßriger Lösung in der Regel eine ungewollte Begrenzung der Haltbarkeit. Diese äußert sich vor allem in einer mit zunehmender Lagerzeit abnehmenden Signalhöhe bei der Messung. Als Konsequenz wird häufig die Haltbarkeitsdauer des gesamten Kits verringert.

Der vorliegenden Erfindung lag somit das technische Problem zugrunde, ein Verfahren bereitzustellen, das die Hydrolyseempfindlichkeit von als Label in immunologischen Nachweisverfahren verwendeten Molekülen herabsetzt.

Aus der EP-0 487 301 war lediglich ein Verfahren zur Stabilisierung von Enzymen bzw. Enzymkonjugaten bekannt, welches die thermische Inaktivierung der Enzyme herabsetzt. Überraschenderweise erfolgt die Lösung des oben genannten durch Hydrolyseempfindlichkeit hervorgerufenen technischen Problems aber durch die Bereitstellung der in den Patentanprüchen gekennzeichneten Ausführungsformen. Es wurde überraschenderweise festgestellt, daß der Zusatz von gegen die zu stabilisierenden Moleküle (Label) gerichteten Antikörpern die Hydrolyseempfindlichkeit der genannten Substanzen beträchtlich herabsetzt. Die Schutzwirkung vor der Hydrolyse beruht vermutlich darauf, daß der Angriff hydrophiler Teilchen (H₂O, OH⁻, H₃O⁺) aus sterischen Gründen und/oder durch Schaffung einer hydrophoben Umgebung erschwert wird. Eine derartige Schutzwirkung vor Hydrolyse erstreckt sich also grundsätzlich auf sämtliche hydrolyseempfindlichen Moleküle (Label), vorausgesetzt, daß gegen diese Substanzen Antikörper erzeugt werden können, um das erfindungsgemäße Verfahren ausführen zu können.

Somit betrifft die Erfindung ein Verfahren zur Stabilisierung von hydrolyseempfindlichen Molekülen in wäßriger Lösung, das dadurch gekennzeichnet ist, daß der wäßrigen Molekül- llösung gegen die Moleküle gerichtete Antikörper zugesetzt werden. Unter dem Begriff "Stabilisierung" ist im Zusammenhang mit der vorliegenden Erfindung eine Verhinderung oder Verlangsamung der Hydrolyse eines Moleküls (Labels) zu verstehen. Unter dem Begriff "Molekül" ("Label")ist eine chemische Verbindung, ausgewählt aus der folgenden Liste von Verbindungen, zu verstehen: Acridiniumester, Acridiniumacylsulfonamid, Luminol, Isoluminol oder dessen Derivate, Dioxetan, Luciferin, Oxalsäureester und Oxalsäureamid. Im Zusammenhang mit der vorliegenden Erfindung fallen auch lonen oder Radikale dieser Moleküle unter diese Bezeichnung. Der Begriff "hydrolyseempfindlich" bezeichnet im Zusammenhang mit der vorliegenden Erfindung die Fähigkeit einer Verbindung, hier eines Moleküls, eines Labels oder Tracers, eine chemische Reaktion unter Beteiligung von H₂O und/oder H₃O⁺ und/oder OH- einzugehen, die zu einem unerwünschten, d.h. einem nicht mehr signalfähigen oder eingeschränkt signalfähigen Produkt führt.

Unter dem Begriff "Antikörper" sind monoklonale oder polyklonale Antikörper zu verstehen. Im Zusammenhang mit der vorliegenden Erfindung fallen auch chimäre Antikörper, bispezifische Antikörper, Antikörperfragmente (z.B. (Fab')₂, Fab, Fv) (semi)synthetische und chemisch modifizierte Antikörper oder Antikörperfragmente unter diesen Begriff.

Das erfindungsgemäße Verfahren ermöglicht eine beträchtliche Erhöhung der Stabilität in wäßriger Lösung von hydrolyseempfindlichen Molekülen, gegen die Antikörper gerichtet werden können und somit eine beträchtliche Erhöhung der Stabilität mit einem Label markierter Tracer, die in immunologischen Nachweisverfahren Verwendung finden.

Die folgenden Beispiele erläutern die Erfindung.

### Beispiel 1

### Stabilisierung eines Anti-PSA-Tracers

### Schritt 1: Herstellung eines monoklonalen Antikörpers gegen ein luminogenes Acridiniumacylsulfonamid-Label

Für die Herstellung monoklonaler Antikörper wurden BALB/c-Mäuse subcutan oder intraperitoneal mit 10 µg Acridiniumacylsulfonamid-BSA injiziert, das in komplettem Freund's Adjuvans emulgiert war. Es folgten 4 bis 5 zusätzliche Immunisierungen ohne Adjuvans (im Abstand von jeweils vier Wochen). Die letzten vier Tage vor der Fusion wurden die Mäuse intravenös nachimmunisiert (10 µg pro Tag).

Zur Herstellung von Hybridomen wurden die immunisierten Tiere mittels cervikaler Luxation getötet. Die Milz wurde aseptisch entfernt und auseinandergezupft, um eine Einzelzellsuspension von Milzzellen in serumfreiem, nach Dulbecco modifiziertem Eagle Medium (DMEM) zu erhalten. Die Zellen wurden mittels Zentrifugation (5 Min.; 1800 UpM.) gesammelt und einmal in DMEM gewaschen. Die Gesamtzellanzahl wurde durch Hämocytometer-Zählung unter Verwendung der Trypanblau-Exklusionstechnik bestimmt. Die als Fusionsparameter verwendeten Maus-Myelomzellen (Sp2/0) wurden zweimal in serumfreiem DMEM gewaschen, mittels Zentrifugation gesammelt (10 Min., 1000 UpM.) und gezählt wie vorstehend für die Milzzellen beschrieben.

Etwa 10⁸ Milzzellen wurden mit 2 x 10⁷ Sp2/O Maus-Myelomzellen gemischt. Nach 10-minütiger Zentrifugation bei 1000 UpM. wurde der Überstand entfernt und 1 ml Polyethylenglycol (PEG 4000, Firma Merck, 50 % ) in das Gefäß mit dem Pellet gegeben. Das Pellet wurde dann unter leichtem Klopfen resuspendiert und 1 Minute bei 37 °C inkubiert 10 ml serumfreies DMEM wurden tropfenweise unter leichtem Klopfen zugegeben und das Gemisch 2 bis 4 Minuten inkubiert. Die fusionierten Zellen wurden anschließend 10 Minuten bei 1000 UpM. zentrifugiert. Das erhaltene Zellpellet wurde in 20 % fötalem Kälberserum (FCS) und HAT (Hypoxanthin 0,1 mM; Aminopterin 0,4 µM; Thymidin 16 µM) enthaltendem DMEM suspendiert und auf Kulturplatten (Nunc) mit 24 Vertiefungen mit einer näherungsweisen Konzentration von 5 x 10⁴ - 10⁶ Zellen pro Vertiefung ausplattiert. Nach 2 bis 3 Wochen wurden einzelne Zellkolonien aus den einzelnen Vertiefungen entnommen und in Vertiefungen einer neuen Kulturplatte kultiviert.

Die Kulturüberstände wurden auf antigenspezifische Antikörper mittels der EIA-Technik abgesucht. Jede mit Acridinium-BSA (3 µg/ml) beschichtete Vertiefung einer Mikrotiterplatte wurde mit 100 µl des Überstandes gefüllt und 1 Stunde bei Raumtemperatur inkubiert. Nach dem Waschen wurden 100 µl eines Kaninchen-Anti-Maus-Antikörper-POD-Konjugats für eine zusätzliche Stunde bei Raumtemperatur zugegeben. Nach 30 Minuten Inkubation mit dem Substrat wurde die Farbentwicklung bei 492 nm auf einem Behring-ELISA-Prozessor (BEP) abgelesen. Hybridome, die Antikörper mit einer geeigneten Antigenspezifität herstellen, wurden ausgewählt und unter Verwendung eines Einzelzellmanipulators kloniert. Zur Herstellung großer Mengen monoklonaler Antikörper wurden die Klone in Massenkultur vermehrt. Die anschließende Reinigung der einzelnen monoklonalen Antikörper wurde mittels Protein A-Chromatographie durchgeführt.

### Schritt 2: Herstellung eines Anti-PSA-Tracers

0,1 mg eines Anti-PSA-MAK (monoklonaler Antikörper, der gegen das Prostataspezifische Antigen (PSA) gerichtet ist; beispielsweise BW 92-284/03, Behringwerke AG) wurden in 0,7 ml 0,1 molarem Phosphatpuffer (pH 8,0) gelöst. Zu dieser Lösung wurden 30 µl einer Markierungslösung (0,1 mg Acridiniumacylsulfonamid (mit N-Succinimidyl-Reaktivgruppe) pro ml Acetonitril) gegeben und die Reaktionslösung 15 Minuten bei Raumtemperatur inkubiert. Anschließend wurden 0,3 ml einer wäßrigen Lysin-Lösung (10 mg/ml) zugegeben und weitere 15 Minuten inkubiert. Der Tracer wurde gelchromatographisch (Sephadex G 25) gereinigt.

Der gereinigte Tracer wurde schließlich in einer Konzentration von 600 ng/ml in einem 0,05 M Tris/HCl Puffer (pH 7,4; 2 g Rinderserumalbumin, 8,8 g NaCl und 0,5 g Natriumazid pro Liter) gelöst.

### Schritt 3: Stabilisierung der Tracer-Lösung

Tracer-Lösung A: Zur Stabilisierung wurden der Tracer-Lösung (Schritt 2) anti-Label-Antikörper (BW 90-9/016, erhalten in Schritt 1, DSM ACC 2183) in einer Konzentration von 1 µg/ml zugesetzt.

Tracer-Lösung B: Bei der Vergleichslösung wurde kein anti-Label-Antikörper zugesetzt.

### Ergebnis

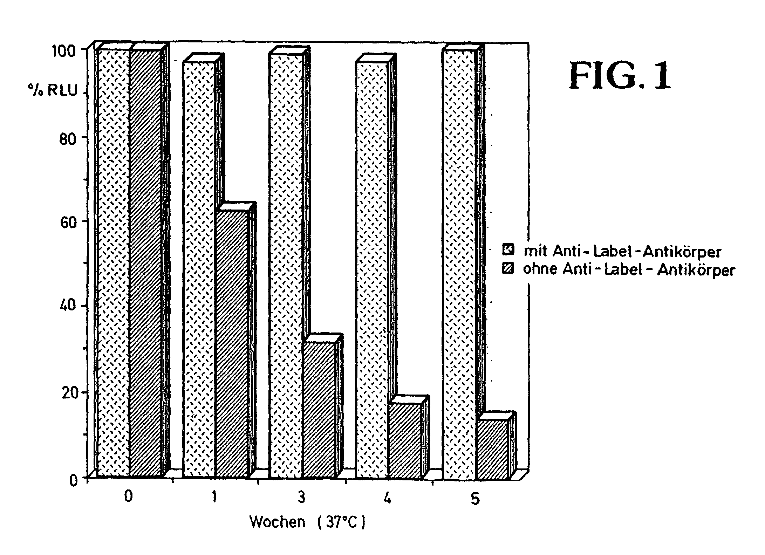

Nach fünfwöchiger Lagerung der Tracer-Lösung A und B bei 37 °C blieb die Anfangssignalaktivität im Falle von Tracer-Lösung A praktisch unverändert und sank im Falle von Tracer-Lösung B um 87 % (Figur 1).

### Beispiel 2

### Stabilisierung eines anti-T3-Tracers

### Schritt 1: Herstellung eines monoklonalen Antikörpers gegen ein luminogenes Acridiniumacylsulfonamid-Label (EP-A-0 257 541 und EP-A-0 330 050)

Der monoklonale Antikörper wurde nach dem in Schritt 1 des Beispiels 1 erläuterten Verfahren hergestellt.

### Schritt 2: Herstellung eines anti-T3-Tracers

0,1 mg anti-T3-MAK, ein gegen das Schilddrüsenhormon Triiodthyronin (T3) gerichteter monoklonaler Antikörper (ImmunoGen International Ltd., Best. Nr. 13-3 D8-38, Gateshead, UK), wurde in 0,7 ml 0,1 molaren Phosphatpuffer (pH 8,0) gelöst. Zu dieser Lösung wurde 5 µl einer Markierungslösung (1,0 mg Acridiniumacylsulfonamid (mit N-Succinimidyl-Reaktivgruppe) pro ml Acetonitril) pipettiert und die Reaktionslösung 15 Minuten bei Raumtemperatur inkubiert. Anschließend wurden 0,3 ml einer wäßrigen Lysin-Lösung (10 mg/ml) zugegeben und weitere 15 Minuten inkubiert. Der Tracer wurde gelchromato-graphisch (Sephadex G 25) gereinigt.

### Schritt 3: Herstellung der Tracer-Lösung

Der gereinigte Tracer (Schritt 2) wurde in einer Konzentration von 90 ng/ml in einem 0,05 M Tris/HCl-Puffer (pH 7,4; 2 g Rinderserumalbumin, 8,8 g NaCl, 0,25 g ANS (8-Anilino-naphthalin-1-sulfonsäure) und 0,5 g Natriumazid pro Liter) gelöst.

### Schritt 4: Stabilisierung der Tracer-Lösung

Tracer-Lösung A: Zur Stabilisierung wurden der Tracer-Lösung (Schritt 3) anti-Label-Antikörper (BW 90-9/016, DSM ACC 2183, Schritt 1) in einer Konzentration von 1 µg/ml zugesetzt.

Tracer-Lösung B: Bei der Vergleichslösung wurde kein anti-Label-Antikörper zugesetzt.

### Ergebnis

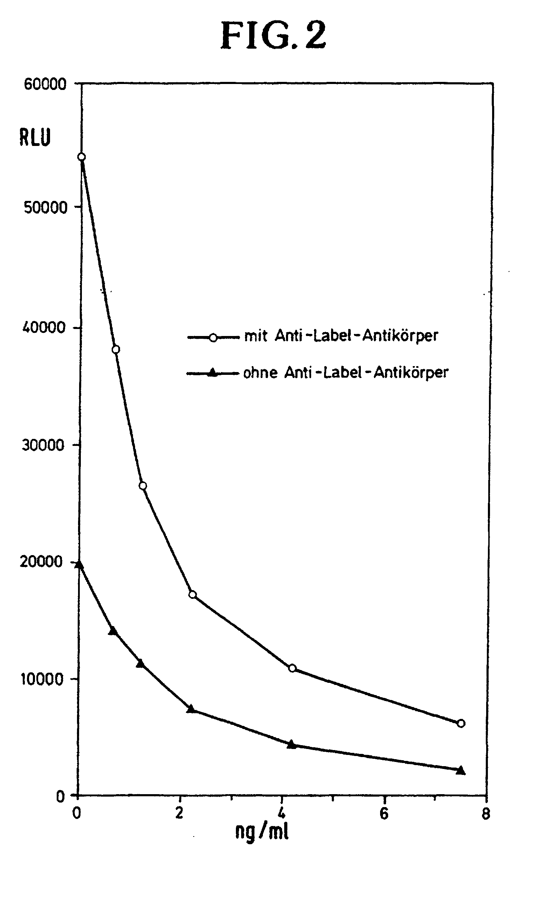

Figur 2 zeigt Standardkurven, die mit den beiden Tracem nach vierwöchiger Lagerung bei 37 °C erhalten wurden. Die Signalhöhe liegt im Falle der Tracer-Lösung A deutlich über der von Tracer-Lösung B.

### Beispiel 3

### Stabilisierung eines T3-Tracers

### Schritt 1: Herstellung eines monoklonalen Antikörpers gegen ein luminogenes Acridiniumsulfonamid-Label (EP-A-0 257 541 und EP-A-0 330 050)

Der monoklonale Antikörper wurde nach dem in Schritt 1 des Beispiels 1 erläuterten Verfahren hergestellt.

### Schritt 2: Herstellung eines T3-Tracers

40 mg Kaninchen-IgG wurden in 8 ml PBS-Puffer, pH 7,2, gelöst. Es wurden 8 ml 0,2 M LiBO₃-Lösung (Wasser mit 20 % Dioxan) und 0,22 ml GMBS-Lösung (10 mg gamma-Maleinimidobutansäure-N-succinimidylester/ml Dioxan) zugesetzt. Nach 1 Stunde bei Raumtemperatur wurde über eine Biogel P2-Säule entsalzt. Das resultierende Eluat ("Lösung 1") hatte ein Volumen von etwa 20 ml.

6,9 mg Triiodthyronin (T3) wurden in 0,69 ml DMSO gelöst. Es wurden 10 µl N-Ethylmorpholin und 2,1 mg SAMSA (S-Acetylmercaptobernsteinsäureanhydrid), gelöst in 0,1 ml DMSO, zugegeben. Nach 30 Minuten wurden 0,1 ml 1 M wäßrige Hydroxylamin-Lösung zupipettiert. Die Lösung ("Lösung 2") wird 15 Minuten bei Raumtemperatur inkubiert. Lösung 1 und Lösung 2 wurden gemischt und 3 ml DMSO zugegeben. Nach 2 Stunden wurde mittels Biogel P6 entsalzt und das Konjugat lyophilisiert.

0,1 mg des IgG-T3-Konjugats wurden in 0,7 ml 0,1 molaren Phosphatpuffer (pH 8,0) gelöst. Zu dieser Lösung wurden 10 µl einer Markierungslösung (0.1 mg Acridiniumacylsulfonamid (mit N-Succinimidyl-Reaktivgruppe) pro ml Acetonitril) pipettiert und die Reaktionslösung 15 Minuten bei Raumtemperatur inkubiert. Anschließend wurden 0,3 ml einer wäßrigen Lysin-Lösung (10 mg/ml) zugegeben und weitere 15 Minuten inkubiert. Der Tracer wurde gelchromatographisch (Sephadex G 25) gereinigt.

### Schritt 3: Herstellung der Tracer-Lösung

Der gereinigte Tracer (Schritt 2) wurde in einer Konzentration von 30 ng/ml in einem 0.05 M Tris/HCl Puffer (pH 7,4; 2g Rinderserumalbumin, 8,8 g NaCI, 0,25 g ANS und 0,5 g Natriumazid pro Liter) gelöst.

### Schritt 4: Stabilisierung der Tracer-Lösung

Tracer-Lösung A: Zur Stabilisierung wurden der Tracer-Lösung (Schritt 3) anti-Label-Antikörper (BW 90-9/016, DSM AACC 2183, Schritt 1) in einer Konzentration von 1µg/ml zugesetzt.

Tacer-Lösung B: Bei der Vergleichslösung wurde kein anti-Label-Antikörper zugesetzt.

### Ergebnis

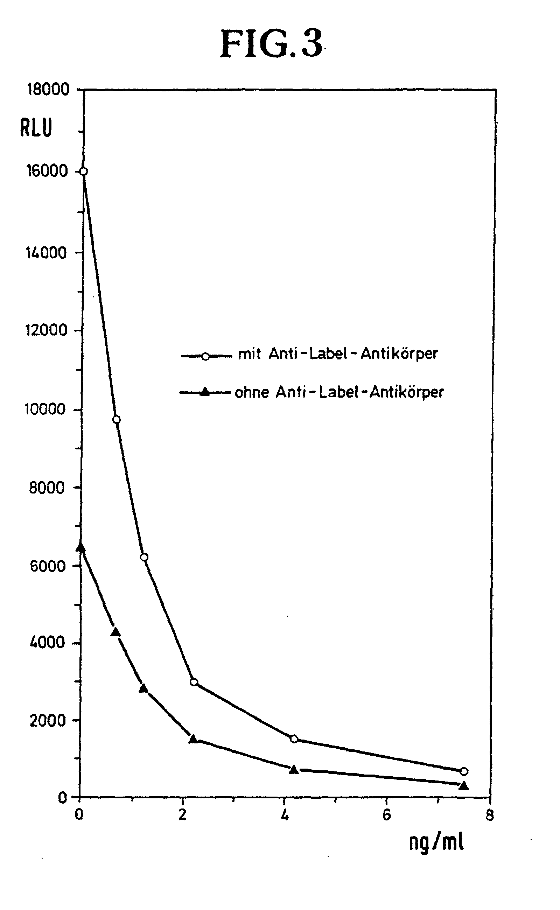

Figur 3 zeigt Standardkurven, die mit den beiden Tracem nach 4-wöchiger Lagerung bei 37 °C erhalten wurden. Die Signalhöhe liegt im Falle der Tracer-Lösung A deutlich über der von Tracer-Lösung B.

### Beispiel 4

### Vergleich verschiedener anti-Label-Antikörper

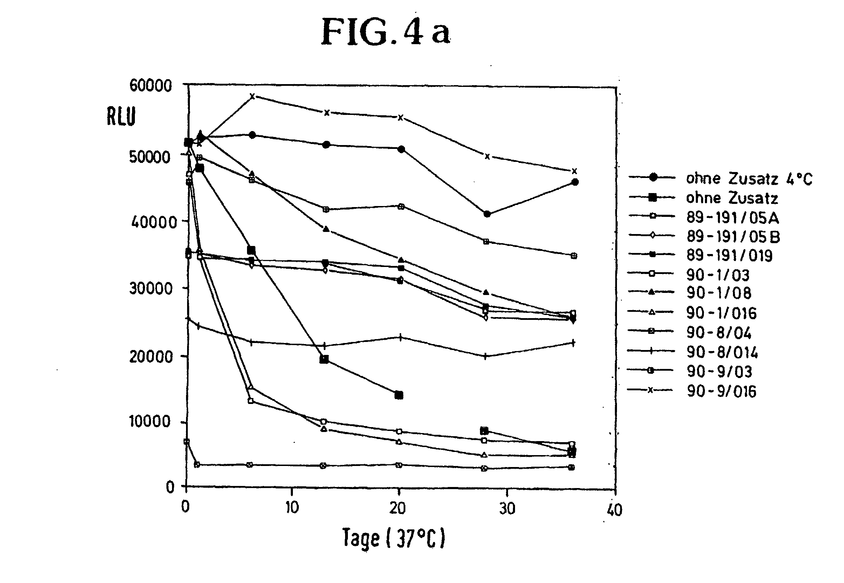

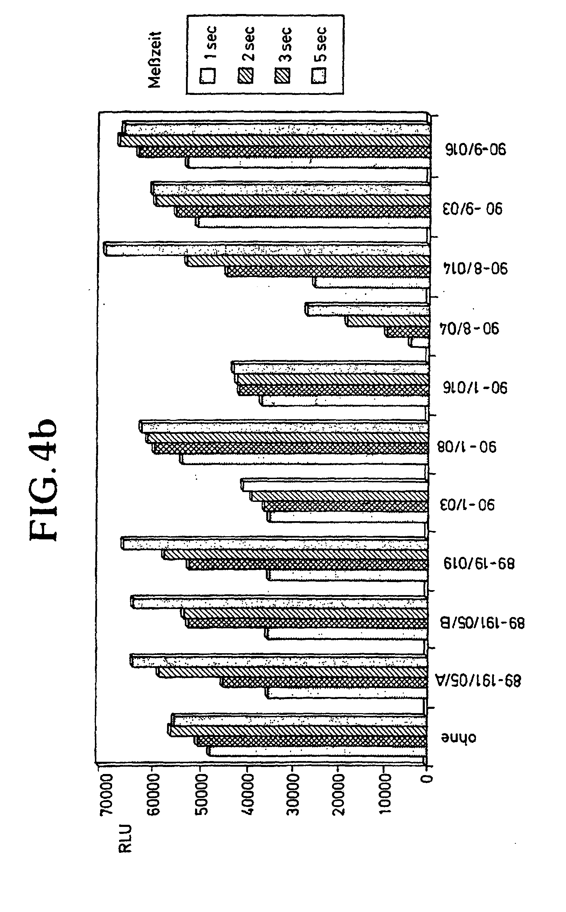

Die untersuchten anti-Labei-Antikörper zeigten zum Teil ein sehr unterschiedliches Verhalten bezüglich ihrer Fähigkeit, Tracer zu stabilisieren (Figur 4a) und bezüglich der Kinetik der Lichtemission. Figur 4b zeigt die Lichtausbeute der verschiedenen anti-Label-Antikörper in Abhängigkeit von der Meßzeit.

Das nachfolgend aufgeführte Hybridom wurden bei der DSM in Braunschweig hinterlegt. Die Bezeichnung des von diesem Hybridom produzierten Antikörpers entspricht der des Hybridoms.

| | | |
|---|---|---|
| **Hybridom** | **DSM-Hinterlegungsnummer** | **Datum der Hinterlegung** |
| BW 90-342-9-016 | 2183 | 21.07.94 |

Die Figuren zeigen:
- Figur 1:: Signaistabilität des anti-PSA-Tracers nach Lagerung bei 37 °C; mit und ohne Zusatz von anti-Label-Antikörpern; RLU bedeutet relative Lichteinheiten.
- Figur 2:: T3-SPALT (solid phase antigen luminescence technique; Festphasen-antigen-Lumineszenzverfahren)-Assays, Durchführung mit 4 Wochen bei 37 °C gelagerten Tracern. Der SPALT-Assay ist ein kompetitiver Lumineszenzimmuntest, bei dem ein markiertes Analytderivat (=Analyttracer) mit dem zu bestimmenden Analyten um die Bindungsstellen eines markierten Antikörpers (Antikörpertracers) konkurriert.
- Figur 3:: T3-LlAs (Lumineszenzimmuntest), Durchführung mit 4 Wochen bei 37 ° C gelagerten Tracern. Der LIA ist ein kompetitiver Lumineszenzim-muntest, bei dem ein markiertes Analytderivat (=Analyttracer) mit dem zu bestimmenden Analyten um die Bindungsstellen eines (hier festphasengebundenen) Antikörpers konkurriert.
- Figur 4a:: Unterschiedliche Stabilisierungswirkung verschiedener anti-Label-Antikörper.
- Figur 4b:: Lichtausbeuten eines anti-PSA-Tracers in Abhängigkeit vom zugesetzten monoklonalen anti-Label Antikörper.

## Patentansprüche

1. Verfahren zur Stabilisierung eines hydrolyseempfindlichen Moleküls in wäßriger Lösung, **dadurch gekennzeichnet, daß** der wäßrigen Moleküllösung ein gegen das Molekül gerichteter Antikörper zugesetzt wird, und daß das Molekül ein in einem immunologischen Nachweisverfahren verwendetes Label ist, ausgewählt aus der folgenden Liste: Acridiniumester, Acridiniumacylsulfonamid, Luminol, Isoluminol oder dessen Derivate, Dioxetan, Luciferin, Oxalsäureester und Oxalsäureamid.

2. Verfahren nach Anspruch 1, **dadurch gekennzeichnet, daß** der Antikörper ein monoklonaler Antikörper, ein polyklonaler Antikörper, ein chimärer Antikörper, ein (semi)synthetischer Antikörper, ein Antikörperfragment oder ein chemisch modifizierter Antikörper oder ein chemisch modifiziertes Antikörperfragment ist.

## Claims

1. A method for stabilizing a hydrolysis-sensitive molecule in aqueous solution, which comprises adding to the aqueous molecular solution an antibody directed against the molecule, and wherein the molecule is a label used in an immunological detection method selected from the following list: acridinium ester, acridiniumacylsulfonamide, luminol, isoluminol or derivatives thereof, dioxetane, luciferin, oxalic ester and oxalic acid amide.

2. The method as claimed in claim 1, wherein the antibody is a monoclonal antibody, a polyclonal antibody, a chimeric antibody, a (semi)synthetic antibody, an antibody fragment or a chemically modified antibody or a chemically modified antibody fragment.

## Revendications

1. Procédé de stabilisation d'une molécule sensible à l'hydrolyse en solution aqueuse, **caractérisé en ce qu'**un anticorps dirigé contre la molécule est ajouté à la solution aqueuse de molécule et que la molécule est un marqueur utilisé dans un procédé de détection immunologique, choisi dans la liste suivante : esters d'acridinium, acylsulfonamide d'acridinium, luminol, isoluminol ou ses dérivés, dioxétane, luciférine, esters d'acide oxalique et amide d'acide oxalique.

2. Procédé selon la revendication 1, **caractérisé en ce que** l'anticorps est un anticorps monoclonal, un anticorps polyclonal, un anticorps chimère, un anticorps (semi)synthétique, un fragment d'anticorps ou un anticorps chimiquement modifié ou un fragment d'anticorps chimiquement modifié.
